# EUROPÄISCHE PATENTSCHRIFT

(11) **EP 0 775 685 B1**
(45) Veröffentlichungstag und Bekanntmachung des Hinweises auf die Patenterteilung: **07.04.1999**
(21) Anmeldenummer: 96118307.6
(22) Anmeldetag: 15.11.1996
(51) Int. Cl.: C07C 45/82, C07C 49/497, C07C 45/61

(54) **Verfahren zur Reindarstellung von trans- und cis-4-Hydroxy-2,2,6-trimethyl-cyclohexan-1-on aus Isomerengemischen**
Process for the isolation of trans- and cis-4-hydroxy-2,2,6-trimethylcyclohexan-1-one from mixtures of these isomers
Procédé d'isolation de la trans- et cis-4-hydroxy-2,2,6-triméthylcyclohexan-1-one à partir de mélanges de ces isomères

(30) Priorität: 23.11.1995 DE 19543619
(43) Veröffentlichungstag der Anmeldung: 28.05.1997
(73) Patentinhaber: BASF AKTIENGESELLSCHAFT, 67056 Ludwigshafen (DE)
(72) Erfinder: Paust, Joachim, Dr., 67141 Neuhofen (DE); Kriegl, Wolfgang, 67071 Ludwigshafen (DE); Hartmann, Horst, 67459 Böhl-Iggelheim (DE)

(56) Entgegenhaltungen:
- EP-A- 0 520 391
- DE-A- 2 537 060
- HELVETICA CHIMICA ACTA, Bd. 59, Nr. 5, 1976, Seiten 1832-1849, XP002023693 LEUENBERGER, H.G.W. ET AL.: "Synthese von optisch aktiven, natürlichen Carotinoiden und strukturell verwandten Naturprodukten. I. Synthese der chiralen Schlüsselverbindung (4R,6R)-4-Hydroxy-2,2,6-trimethylcyclohexa non"

## Beschreibung

Die Erfindung betrifft ein auch in industriellem Maßstab anwendbares Verfahren zur Reindarstellung von trans-4-Hydroxy-2,2,6-trimethyl-cyclohexan-1-on der Formel I und cis-4-Hydroxy-2,2,6-trimethyl-cyclohexan-1-on der Formel II aus Gemischen dieser Isomeren.

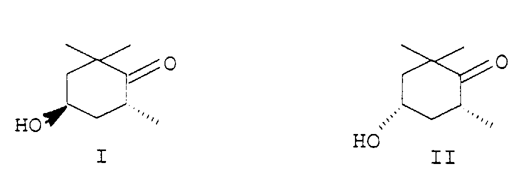

Carotinoide mit chiralen alkoholischen Hydroxylgruppen kommen in der Natur relativ häufig als natürliche Farbstoffe vor. Genannt seien beispielsweise Cryptoxanthin der Formel 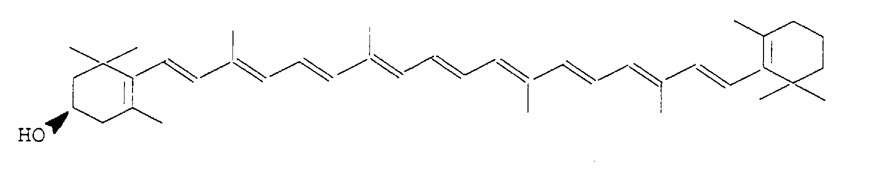 und Zeaxanthin der Formel

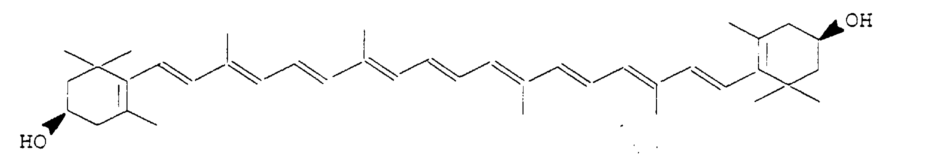

Zeaxanthin ist beispielsweise die hauptsächliche Farbstoffkomponente des gelben Mais, die als natürlicher Lebensmittelfarbstoff sehr begehrt ist. Die natürlichen 3-Hydroxy-Carotinoide sind optisch aktiv. Es hat daher nicht an Versuchen gefehlt, ein Verfahren zur Herstellung von optisch aktiven 3-Hydroxy-Carotinoiden zu entwickeln (vgl. Helv. Chim. Akta, Vol 73 (1990), S. 861-67). Essentielle Zwischenprodukte für solche Synthesen sind die trans- oder cis-4-Hydroxy-2,2,6-trimethyl-cyclohexan-1-one der Formeln I und II. Ein Gemisch beider Isomeren erhält man recht gut bei der Reduktion des 1,3-Diketons der Formel III 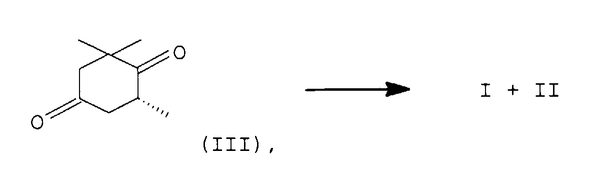 welches seinerseits aus Oxo-isophoron erhältlich ist.

Für die Trennung des Gemisches der Isomeren I und II sind zwar einige Verfahren bekannt, jedoch ist keins bislang in technischem Maßstab praktikabel.

So ist beispielsweise aus Helv. Chim. Acta 59 (1976), Seiten 1832-49, bes. 1844, bekannt, das aus dem Ausgangsketon und den Isomeren I und II bestehende Gemisch durch präparative Säulenchromatographie an Kieselgel oder durch vielstufige Gegenstromverteilung in einer Craig-Apparatur zu trennen.

Weiterhin ist aus DE 2 537 060 bekannt, das bei der Reduktion von [R]-2,2,6-Trimethyl-1,4-cyclohexandion (III) anfallende Gemisch nach aufwendiger Vorreinigung durch Chromatographie an Kieselgel mit n-Hexan/Diethylether als Eluierungsmittel und anschließende Kristallisation bei -45 bis -70°C aufzutrennen.

Weiterhin ist aus EP 520 391 bekannt, ein Gemisch der Isomeren der Formeln I und II dadurch zu trennen, daß man das Gemisch mit einer Verbindung umsetzt, durch die im wesentlichen nur der Wasserstoff der Hydroxylgruppe von der trans-Verbindung der Formel I substituiert wird und aus dem Gemisch der modifizierten trans-Verbindung und der cis-Verbindung letztere aufgrund der unterschiedlichen Löslichkeiten in Wasser oder organischen Lösungsmitteln isoliert.

Da die bekannten Trennverfahren für ein vorteilhaftes Arbeiten in industriellem Maßstab wenig geeignet sind, war es Aufgabe der Erfindung, ein Verfahren zur Trennung von trans- und cis-4-Hydroxy-2,2,6-trimethyl-cyclohexan-1-on zu finden, das auch in industriellem Maßstab auf vorteilhafte Weise durchgeführt werden kann.

Gegenstand der Erfindung ist dementsprechend ein Verfahren zur Reindarstellung von trans- und cis-4-Hydroxy-2,2,6-trimethyl-cyclohexan-1-on der Formeln I und II 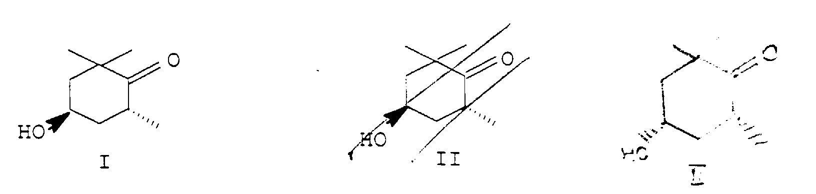 aus Gemischen dieser Isomeren, das dadurch gekennzeichnet ist, daß man das Isomerengemisch in einer geeigneten Kolonne mit ca. 30 bis 80, vorzugsweise 50 bis 60, theoretischen Trennstufen bei Temperaturen von 50 bis 130°C, vorzugsweise 70 bis 110°C, in einem Druckbereich von 0,1 bis 5 mbar, vorzugsweise 0,5 bis 2,5 mbar fraktionierend rektifiziert.

Daß die Trennung der Isomeren I und II durch fraktionierende Rektifikation so vorteilhaft gelingt war sehr überraschend, da in Helv. Chim. Acta, Vol. 59, Fasc. 5 (1976), Seite 1845 ausdrücklich auf die Empfindlichkeit des trans-4-Hydroxy-2,2,6-trimethyl-cyclohexan-1-ons gegenüber sauren oder basischen Bedingungen sowie höheren Temperaturen hingewiesen wird. Diese Empfindlichkeit wird durch Äquilibrierung der Methylgruppe in Position 6 erklärt.

Das als Ausgangsstoff eingesetzte Gemisch der Isomeren I und II erhält man in an sich bekannter Weise durch Reduktion von (6R)-2,2,6-Trimethyl-1,4-cyclohexandion (III), welches seinerseits durch Hefereduktion von Oxo-isophoron erhältlich ist.

Für die erfindungsgemäße Rektifikation im Feinvakuum eignen sich neben Füllkörperkolonnen mit geringem Druckverlust insbesondere Kolonnen mit geordneten Gewebepackungen, wie Feingewebepackungen nach Kloss (Montz GmbH), Sulzer-Packungen (Sulzer AG) vom Typ DX, CY oder BX oder aber ähnliche Packungen anderer Firmen. Besonders vorteilhaft gelingt die Rektifikation an Sulzer-Packungen vom Typ Sulzer DX.

Die Rektifikation kann diskontinuierlich und kontinuierlich vorteilhaft durchgeführt werden.

Bei der diskontinuierlichen Durchführung besteht die Apparatur im wesentlichen aus einer beheizten Blase, einer geeigneten Trennkolonne und einem beheizten Kondensator, der mit einem Flüssigkeitsteiler ausgerüstet ist.

Bei der kontinuierlichen Durchführung besteht die Apparatur im wesentlichen aus einer oder auch mehreren kontinuierlich betriebenen Rektifizieranlagen mit Verstärkungskolonne und Abtriebskolonne, wie sie beispielsweise in dem Lehrbuch "Thermische Trennverfahren" von Klaus Sattler, VCH-Verlagsgesellschaft mbH, Weinheim, 1988, Seite 103, schematisch dargestellt wird.

Für eine vorteilhafte Durchführung des Rektifikationsverfahrens ist es wichtig, daß man die Rektifikation adiabatisch betreibt, d. h. dafür sorgt, daß praktisch kein Wärmeverlust durch die Kolonnenwand auftreten kann. Deshalb ist es geboten, daß die Kolonne mit einer Kombination aus Isolierung und Schutzheizung versehen wird, die jeglichen Abfluß von Wärme aus der Kolonne verhindert. Für kleinere Anlagen eignet sich hierzu z.B. ein verspiegelter Vakuum-Mantel kombiniert mit einer sogenannten Heizjacke.

Die technische Ausführung einer solchen Kombination aus Isolierung und Schutzbeheizung wird mit Vorteil praktisch auf folgende Weise realisiert: Auf einer ersten Isolierschicht auf dem Kolonnenmantel wird ein Blechmantel angebracht. Dieser Blechmantel wird erneut isoliert. Auf diese Isolierschicht wird dann ein weiterer Blechmantel und die Heizung aufgebracht und diese schließlich nach außen isoliert. Die Regelung der Heizung erfolgt dann derart, daß die Temperaturdifferenz zwischen dem Kolonnenmantel und dem ersten Blechmantel Null beträgt.

Da die Isomeren bei etwa 60°C beginnen zu kristallisieren, ist es weiterhin wichtig, daß die produktführenden Leitungen und der Kondensator während der Rektifikation auf Temperaturen oberhalb des Kristallisationspunktes, d.h. oberhalb von 60°C erwärmt werden.

Für eine vorteilhafte Verfahrensführung ist es insbesondere beim kontinuierlichen Arbeiten vorteilhaft, wenn man die Flüssigkeit in der Kolonne gut und schnell verteilt. Besonders geeignet sind für die Flüssigkeitsverteilung in der Kolonne sogenannte Kanalverteiler mit möglichst vielen Abtropfstellen pro m², beispielsweise mit 500 bis 1000 Abtropfstellen pro m².

Mit Hilfe des erfindungsgemäßen Verfahrens können sowohl Gemische, die im wesentlichen die Enantiomeren (4R, 6R)- und (4S,6R)-4-Hydroxy-2,2,6-trimethyl-cyclohexan-1-on enthalten, als auch Gemische, die im wesentlichen racemisches trans- und cis-4-Hydroxy-2,2,6-trimethyl-cyclohexan-1-on enthalten, vorteilhaft getrennt werden. Die Trennung und die Ausbeuten sind praktisch quantitativ. Hierdurch wird es möglich, die 3-Hydroxy-Cryptoxanthin und Zeaxanthin sowohl in optisch aktiver Form als auch in racemischer Form technisch vorteilhaft herzustellen.

Die folgenden Beispiele sollen das erfindungsgemäße Verfahren näher erläutern.

### Beispiel 1

Verwendet wurde eine Apparatur zur fraktionierenden Rektifikation, bestehend aus einem 1-Liter-Kolben, einer Kolonne von 1,90 m Länge und 30 mm Durchmesser, gefüllt mit einer Sulzer DX-Packung (entsprechend ca. 30 - 35 Trennstufen) und einem mit Flüssigkeitsteiler ausgerüsteten Kondensator. Der Kolben wurde mit einem Ölbad beheizt. Kolben und Ölbad wurden magnetisch gerührt. Die Kolonne war mit einem verspiegelten Vakuum-Mantel und einer sogenannten Heizjacke versehen, die eine adiabatische Verfahrensführung ermöglicht.

Die Untersuchung der eingesetzten Gemische und der erhaltenen Produkte erfolgte mit Hilfe von H-NMR-Spektroskopie [Hewlett-Packard Ser. II bei 130°C (10 Minuten) → 230°C (10°C pro Minute), 15 PSI, WCOT-Fused Silicon 25 m x 0,25 mm (Chrompack), Coating CP-Chirasil-DFX CB, DF 0,25].

456 g eines Gemisches enthaltend 40 % 4R,6R-4-Hydroxy-2,2,6-trimethyl-cyclohexan-1-on (I) und 60 % seines 4S,6R-Isomeren (II) wurden in der oben beschriebenen Apparatur bei einem Druck von 0,4 mbar (Übergang) und 4 mbar Differenzdruck mit einem Rücklauf-Verhältnis von 40/1 bei Temperaturen von 110°C im Kolben, 78 bis 81°C beim Übergang und 45 bis 50°C im Kondensator fraktionierend rektifiziert.

Folgendes Ergebnis wurde erzielt:
1) Vorlauf: 6 g
2) Destillat I: 90 g, enthaltend 99 % 4R,6R-I
3) Zwischenlauf:130 g, enthaltend 70 % 4R,6R-I und 30 % 4S,6R-II
4) Destillat II: 236 g, enthaltend 92 % 4S,6R-II und höhersiedende Nebenprodukte.

Der noch ein Gemisch enthaltende Zwischenlauf kann wieder in den Rektifikationsprozess zurückgeführt werden.

Auf diese Weise erzielt man letztlich eine praktisch quantitative Trennung mit nahezu quantitativen Ausbeuten.

### Beispiel 2

In der in Beispiel 1 beschriebenen Apparatur wurden im wesentlichen unter den in Beispiel 1 beschriebenen Bedingungen 578 g eines Gemisches enthaltend 2 % Leichtsieder, 74 % racemisches trans-4-Hydroxy-2,2,6-trimethyl-cyclohexan-1-on, 23 % seines racemischen cis-Isomeren sowie 1 % Hochsieder fraktionierend rektifiziert.

Folgendes Ergebnis wurde erzielt:
- Destillat I:: 370 g enthaltend 85 % des racemischen trans-4-Hydroxy-2,2,6-trimethyl-cyclohexan-1-on und 15 % Leichtsieder
- Zwischenlauf:: 100 g enthaltend 60 % racemisches trans-4-Hydroxy-2,2,6-trimethyl-cyclohexan-1-on und 40 % seines racemischen cis-Isomeren
- Destillat II: 75 g enthaltend mehr als 90 % des racemischen cis-4-Hydroxy-2,2,6-trimethyl-cyclohexan-1-on.

## Patentansprüche

1. Verfahren zur Reindarstellung von trans-4-Hydroxy-2,2,6-trimethyl-cyclohexan-1-on der Formel I und cis-4-Hydroxy-2,2,6-trimethyl-cyclohexan-1-on der Formel II 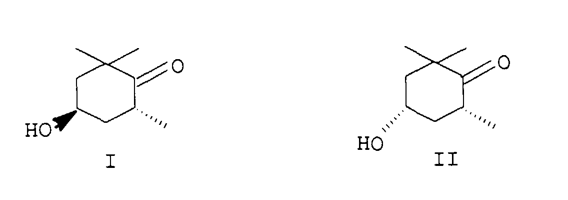 durch Trennung eines Gemisches dieser Isomeren, dadurch gekennzeichent, daß man das Isomerengemisch in einer Füllkörperkolonne mit geringem Druckverlust oder einer Kolonne mit geordneten Gewebepackungen, die ca. 30 bis 80 theoretische Trennstufen aufweisen, bei Temperaturen von 50 bis 130°C im Druckbereich von 0,1 bis 5 mbar fraktionierend rektifiziert.

2. Verfahren gemäß Anspruch 1, dadurch gekennzeichnet, daß man als geeignete Kolonne eine Kolonne mit sogenannten geordneten Gewebepackungen verwendet.

3. Verfahren gemäß Anspruch 1, dadurch gekennzeichnet, daß man als geeignete Kolonne eine Kolonne mit ca. 50 bis 60 theoretischen Trennstufen verwendet.

4. Verfahren gemäß Anspruch 1, dadurch gekennzeichnet, daß man mit einer Rektifikationsapparatur arbeitet, die so mit Schutzheizungen versehen ist, daß
a) während der Rektifikation praktisch kein Wärmeverlust durch die Kolonnenwand auftreten kann
und
b) die produktführenden Rohrleitungen und der Kondensator auf Temperaturen oberhalb des Kristallisationspunktes der Komponenten erwärmt werden.

## Claims

1. A process for the preparation of pure trans-4-hydroxy-2,2,6-trimethylcyclohexan-1-one of the formula I and cis-4-hydroxy-2,2,6-trimethylcyclohexan-1-one of the formula II 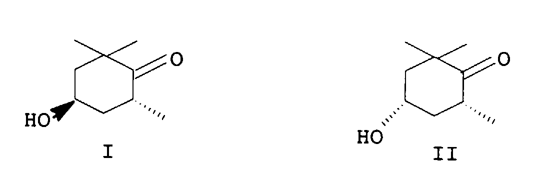 by separating a mixture of these isomers, which comprises fractionally rectifying the isomeric mixture in a dumped packing column with low drop of pressure or a column with arranged woven gauze packing which have from about 30 to 80 theoretical separation stages at temperatures from 50 to 130°C and a pressure in the range from 0.1 to 5 mbar.

2. A process as claimed in claim 1, wherein the column used is packed with arranged woven gauze packing.

3. A process as claimed in claim 1, wherein the column used has from about 50 to 60 theoretical separation stages.

4. A process as claimed in claim 1, wherein the rectification apparatus used is equipped with protective heating in such a way that
a) virtually no heat can be lost through the column wall during the rectification
and
b) the product-carrying pipework and the condenser are heated to temperatures above the crystallization point of the components.

## Revendications

1. Procédé pour préparer à l'état pur la trans-4-hydroxy-2,2,6-triméthyl-cyclohexan-1-one de formule I et la cis-4-hyroxy-2,2,6-triméthyl-cyclohexan-1-one de formule II 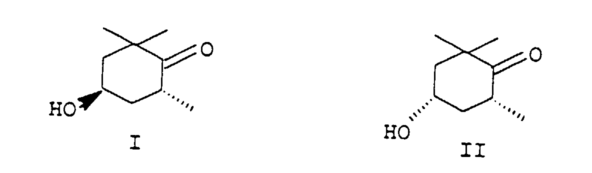 par séparation d'un mélange de ces isomères, caractérisé par le fait que l'on soumet le mélange d'isomères à rectification fractionnée dans une colonne à corps de garnissage à faible perte de pression ou dans une colonne à garnissage de tissu ordonné, à environ 30 à 80 étages de séparation théoriques, à des températures de 50 à 130°C, dans l'intervalle de pression de 0,1 à 5 mbar.

2. Procédé selon la revendication 1, caractérisé par le fait que l'on utilise, en tant que colonne appropriée, une colonne dite à garnissage de tissu ordonné.

3. Procédé selon la revendication 1, caractérisé par le fait que l'on utilise, en tant que colonne appropriée, une colonne à environ 50 à 60 étages de séparation théoriques.

4. Procédé selon la revendication 1, caractérisé par le fait que l'on opère avec un appareil de rectification équipé de chauffages protecteurs assurant que
a) il ne peut pratiquement pas se produire de perte de chaleur au travers des parois de la colonne en cours de rectification
et
b) les conduits transportant les produits et le condenseur sont chauffés à des températures supérieures au point de cristallisation des composants.
